# FASCICULE DE BREVET EUROPEEN

(11) **EP 0 817 641 B1**
(45) Date de publication et mention de la délivrance du brevet: **04.06.2003**
(21) Numéro de dépôt: 97901124.4
(22) Date de dépôt: 20.01.1997
(51) Int. Cl.: A61K 35/80, A23L 1/337, A01K 61/00, A61P 17/02

(54) **SUBSTANCES EXTRAITES DE DICTYOTALES, LEUR PROCEDE D'OBTENTION ET LES COMPOSITIONS EN RENFERMANT**
AUS DICTYOTALES EXTRAHIERTE SUBSTANZEN, IHR HERSTELLUNGSVERFAHREN UND SIE ENTHALTENDE ZUSAMMENSETZUNGEN
SUBSTANCES EXTRACTED FROM DICTYOTALES, PREPARATION METHOD THEREFOR, AND COMPOSITIONS CONTAINING SAME

(30) Priorité: 18.01.1996 FR 9600522
(43) Date de publication de la demande: 14.01.1998
(73) Titulaire: TEXINFINE, 69466 Lyon Cédex 06 (FR); LABORATOIRE LAPHAL, 13190 Allauch Cédex (FR)
(72) Inventeur: GUTIERREZ, Gilles, F-69006 Lyon (FR)
(74) Mandataire: Burtin, Jean-François
(86) Numéro de dépôt international: FR9700106
(87) Numéro de publication internationale: WO97025998

(56) Documents cités:
- EP-A- 0 655 250
- BRITISH PHYCOLOGICAL JOURNAL, vol. 21, no. 2, Juin 1986, pages 217-224, XP000601600 MEGUMI OKAZAKI ET AL.: "A STUDY OF CALCIUM CARBONATE DEPOSITION IN THE GENUS PADINA (PHAEOPHYCEAE, DICTYOTALES)"

## Description

La présente invention se rapporte au domaine de la biologie, et plus précisément à des substances biologiquement actives d'origine marine.

La présente invention concerne de nouvelles substances biologiquement actives extraites de plantes de la famille des Dictyotaceae, leur procédé d'obtention et les compositions en renfermant.

Les compositions de la présente invention sont destinées à remanier la synthèse des éléments glycosylés de la Matrice Extra Cellulaire (MEC) des tissus animaux et humains.

Par le terme plantes de la famille des Dictyotaceae, il faut comprendre plus particulièrement celles susceptibles de fixer, condenser, précipiter ou d'effectuer sur leur thalle la synthèse du carbonate de calcium sous la forme cristalline dite aragonite.
Le terme de plantes peut concerner la plante sèche ou fraîche, broyée ou brute.

Les plantes desquelles sont extraites les substances biologiquement actives de l'invention, sont des Phéophycées, classe des Fucophyceae, ordre des Dictyotales, famille des Dictyotaceae. Seuls quelques genres appartiennent à la famille des Dictyotaceae, il s'agit de Padina, Zonaria ou Dictyota. Le genre Padina regroupe les espèces les plus fréquentes. A titre d'exemple, il est possible de trouver sur le rivage de la Méditérranée les espèces pavonica, boyana (P. tenuis), boergesenii, sur les rives du Pacifique (non repris les espèces déjà citées) : arborescens, australis, boryana, caulens, commersonii, concrescens, crasse, durvillei, elegans, fernandeziana, fraseri, gymnospora, de même sur les rives de l'Océan Atlantique : glabra, haitensis, distromatica, dubia. Il existe aussi des espèces typiques de l'océan indien. L'une des caractéristiques de ces plantes est de fixer sur leur thalle une couche de carbonate de calcium de type aragonite ou orthorhombique à la surface des frondes. Cette caractéristique est obtenue par l'examen en diffraction X de la poudre obtenue avec ces plantes. La diffraction des rayons X montre que la poudre de ces plantes présente des pics d'intensité notable aux angles 2 θ : -3,393°, -3,268°, -2,699°, - 2.682°. - 2,371°, -2,336° (doublet), - 1,976°, - 1, 877° (doublet). Ces angles de diffraction sont caractéristiques de l'aragonite.

Le document EP 0 655 250 décrit des compositions à base d'algues Padina ou de leurs extraits obtenues par séchage des algues Padina pavonica, suivi de broyage et suspension dans l'éthanol pur.

La présente invention a pour objet de nouvelles substances biologiquement actives, remaniant la synthèse des éléments glycosylés de la matrice extracellulaire des tissus animaux et humains, extraites de plantes de la famille des Dictyotaceae.
Ces nouvelles substances biologiquement actives, sont notamment caractérisées par une structure polycyclique portant une chaîne latérale ayant de 4 à 10 atomes de carbone en ligne droite ou ramifiée et comportant une où deux doubles liaisons.

Les nouvelles substances sont encore caractérisées par des données analytiques plus précises.

La présente invention concerne encore un procédé d'obtention des substances biologiquement actives extraites de plantes de la famille des Dictyotaceae
Dans un premier temps, la plante de la famille des Dictyotaceae est soumise, après récolte, à un séchage, si possible à l'abri de la lumière, pour garder intactes ses propriétés pharmacologiques.
Un séchage lent avec une insolation importante conduit à l'obtention de substances peu actives et instables. De ce fait, on utilise de préférence un séchage selon des méthodes plus appropriées, avec pour principale condition que l'on obtienne des substances actives.
Pour cette raison, on procède au séchage des plantes broyées ou brutes, par ventilation sans chauffage et on obtient ainsi un matériel végétal de couleur marron foncé sur lequel contrastent vivement les stries blanches de carbonate de calcium.
Une autre méthode, bien que moins économique, donne également satisfaction : elle consiste à égoutter fortement les plantes, puis à les sécher sous vide d'air. La lyophilisation de la plante, broyée ou non, permet d'une manière avantageuse d' obtenir un produit anhydre.
Ces deux dernières techniques donnent un matériel de couleur vert brun foncé, très fragile et facilement réduit en poudre, ce qui facilite l'extraction par un solvant pour obtenir les substances biologiquement actives.

L'intérêt d'une plante fortement séchée est d'obtenir une matière végétale qui se conserve bien (à l'abri de l'humidité) et à partir de laquelle il sera possible d'extraire la ou les substances actives, stabilisée(s) ou non, sans être gêné par la présence d'un solvant polaire. De plus, les plantes complètement séchées sont plus faciles à réduire en poudre.

Le procédé d'obtention des substances biologiquement actives est caractérisé en ce que l'on soumet la plante de la famille des Dictyotaceae à un séchage, et/ou à une lyophilisation, à un broyage, suivi d'un ou plusieurs épuisement de la matière végétale par un solvant organique choisi dans le groupe formé des alcanols inférieurs (éthanol...), des cétones aliphatiques (acétone...), des alcanes (pentane, hexane, heptane...), des cycloalcanes (cyclohexane...), des solvants halogénés (chloroforme, dichlorométhane...), des solvants aromatiques, des esters (acétate d'éthyle...), des éthers et similaires, pour obtenir un extrait organique de la plante, on sèche l'extrait organique par évaporation du solvant, on purifie l'extrait organique sec par une ou plusieurs étapes de purification réalisées successivement par affrontement liquide/liquide, par chromatographie sur colonne basse pression ou haute pression, par chromatographie liquide haute performance, pour obtenir les substances actives des plantes de la famille des Dictyotales.

Les solvants organiques peuvent être utilisés seul ou en association. De manière générale, on peut utiliser des solvants organiques miscibles à l'eau, ainsi que tous les solvants susceptibles d'extraire les substances actives des frondes de la plante.
Les solvants seront de préférence encore choisis parmi les solvants volatils dès lors que l'on désire obtenir des substances biologiquement actives débarrassées de solvant. En effet, les solvants volatils dissolvant les substances biologiquement actives pourront être évaporés à partir d'un support comme de la cellulose ou ses dérivés, de la silice colloïdale, des alcanes de haut poids moléculaire tels des paraffines, des solvants amphiphiles comme le polyéthylène glycol (PEG), le propylène glycol, ou encore de produits inertes comme le glycérol etc.

Le solvant organique sera de préférence, l'acétone ou l'éthanol.
Le rapport utilisé est de préférence de 1 gramme de matière végétale sèche pour 5 ml de solvant et, les temps de contact entre la matière végétale et le solvant organique sont de préférence de 4 à 5 jours.

Néanmoins, lors de la macération, on a utilisé, avec de bons résultats, des rapports très variables de matière sèche par rapport au solvant. Les volumes mis en oeuvre pour une même quantité de matière sèche varient de 1 à 50. De même, les temps de contact entre le solvant organique et la matière végétale varient de 12 heures à 5 jours.

De plus, bien que plus délicate à mettre en oeuvre, l'extraction aqueuse en milieu ammoniacal donne des résultats satisfaisants.
Pour réaliser l'extraction des substances biologiquement actives, il n'est pas nécessaire de faire macérer les matières végétales dans le solvant choisi, il est possible de faire passer en continu le solvant sur la poudre (percolation). Il suffit de régler le débit et de concentrer l'extrait pour obtenir une solution d'activité choisie.

Dans le cas présent, la plante de la famille des Dictyotales une fois séchée, et/ou lyophylisée et broyée, est soumise à un ou plusieurs épuisements par la mise en contact de la plante avec de l'acétone, pendant 4 à 5 jours et dans les proportions de 1 gramme de matière pour 5 ml d'acétone.
L' extrait acétonique obtenu est séché par évaporation du solvant, puis purifié par les étapes de purification définies ci-après.

La première étape de purification est réalisée par affrontement liquide/liquide. Le résidu ou extrait acétonique sec est repris par un mélange méthanol/eau qui est affronté à de l'hexane. Dans un deuxième temps, la phase hydrométhanolique est affrontée, après concentration et dilution dans l'eau, à l'éther.
De cette manière, la phase éthérée finale est biologiquement active.

La deuxième étape de purification est réalisée par chromatographie sur colonne basse pression. Le support utilisé est un gel de type Sephadex LH 20 ®.
L'élution s'effectue par un gradient chloroforme/méthanol. La fraction biologiquement active est éluée en chloroforme/méthanol dans les proportions 97/3 volume à volume. Lors du rinçage de la colonne par un mélange chloroforme/méthanol 70/30, on élue une deuxième fraction active résiduelle.

Les étapes suivantes de purification ont lieu par chromatographie liquide haute performance (HPLC) semi préparative. Les dimensions de la colonne sont de 250 mm (pour la longueur) et de 10 mm (pour le diamètre). Les supports utilisés sont : une silice greffée C₁₈ et une silice greffée diol. Le débit est de 8 ml/min.
Sur le support silice greffée diol, avec un éluant hexane/isopropanol 92/8, les fractions actives sont éluées entre 20 et 25 min et 35 et 40 min.
Sur le support silice greffée C₁₈, avec un éluant acétonitrile/eau 85/15, la fraction active est éluée entre 15 et 17 min, et avec un éluant acétonitrile/eau/acide acétique 85/15/0,05, les fractions actives sont éluées entre 17 et 20 min et 30 et 40 min.

A l'issue de ces étapes de purification, on obtient une substance biologiquement active de couleur jaune-verte.
On procède à différentes méthodes analytiques (spectres UV, IR, RMN, chromatographie sur couche mince...) de caractérisation de la ou des substances biologiquement actives, extraites de plantes de la famille des Dictyotaceae.

Le spectre UV de la fraction active finale, réalisé dans le méthanol est caractérisé par plusieurs bandes d'absorption situées à 202 nm avec un épaulement à 228 nm, entre 260 et 270 nm, entre 400 et 460 nm, et, à 630 nm.

A l'issue de ces étapes de purification, on réalise encore une chromatographie sur couche mince (CCM) avec révélation chimique (les révélateurs utilisés sont l'anisaldéhyde sulfurique, la vanilline sulfurique, le phosphomolybdate, l'acide sulfurique éthanolique). Après révélation, on note la présence de plusieurs tâches. Les temps de rétention frontale sont : Rf = 0,15 et 0,90, 0,95, et correspondent à des zones biologiquement actives issues d'une CCM préparative réalisée dans les mêmes conditions, à savoir, plaque de CCM avec silice greffée diol, élution par hexane/isopropanol 85/15.

Les spectres de masse, les spectres IR et les spectres RMN du proton ou du carbone 13, ont également permis de compléter les informations quant à la structure des nouvelles subtances biologiquement actives extraites de plantes de la famille des Dictyotales selon l'invention.
L'analyse du spectre RMN du carbone 13 démontre notamment la présence de la chaîne latérale ayant de 4 à 10 atomes de carbone, en ligne droite ou ramifiée et comportant une à deux doubles liaisons, portée par la structure polycyclique.

Les substances biologiquement actives extraites de plantes de la famille des Dictyotaceae, ont également pu être obtenues par macération ou lixiviation de 1g de matière végétale sèche dans 5 ml d'éthanol pur pendant 12 heures.
L'éthanol est un solvant bien accepté par les cellules et il est entièrement miscible aux solutions nutritives aqueuses dans lesquelles baignent les cellules.
Les étapes suivantes de purification ont ensuite été réalisées.
On réalise une chromatographie haute pression sur une colonne de silice normale. On injecte 20 µl de la fraction biologiquement active dans un chromatographe haute pression puis élue la fraction avec un mélange heptane/éther (90/10) sous un débit de 2 ml/min. Toute l'activité des substances extraites des plantes de la famille des Dictyotales se retrouve dans la fraction éluée entre 6,9 min et 8,5 min.
Il est possible de procéder de la même façon par chromatographie liquide haute performance (HPLC). La colonne d'HPLC est garnie avec une silice greffée en C₁₈, l'éluant est un mélange méthanol/eau 80/20, et le débit est de 4 ml/min. Toute l'activité des substances extraites est alors concentrée dans la fraction éluée après un temps de rétention compris entre 16 et 18 min.

Le chromatogramme réalisé avec un détecteur à diffusion de lumière montre, à ce temps de rétention, une certaine quantité de substance qui constitue la fraction active de la plante à condition de travailler sous une pression réduite, de telle manière que l'évaporation de la phase mobile ne se fasse pas avec entraînement de la fraction active.

Ainsi, les nouvelles substances biologiquement actives extraites de plantes de la famille des Dictyotaceae sont donc caractérisées lors des étapes de purification, et lorsque les épuisements ont été effectués à l'acétone, par les données analytiques suivantes :
- des temps de rétention, en chromatographie liquide haute performance semi-préparative sur le support silice greffé diol et avec un éluant hexane/isopropanol 92/8, compris entre 20 et 25 min et 35 et 40 min.
- un temps de rétention, en chromatographie liquide haute performance semi-préparative sur le support silice greffée C₁₈ et avec un éluant acétonitrile/eau 85/15, compris entre 15 et 17 min.
- des temps de rétention, en chromatographie liquide haute performance semi-préparative sur le support silice greffée C₁₈ et avec un éluant acétonitrile/eau/acide acétique 85/15/0,05, compris entre 17 et 20 min et 30 et 40 min.

Lors des étapes de purification et lorsque les épuisements ont été effectués à l'éthanol, les données analytiques sont :
- un temps de rétention, en chromatographie haute pression sur colonne de silice normale et avec un éluant heptane/éther (90/10), compris entre 6,9 min et 8,5 min.
- un temps de rétention, en chromatographie liquide haute performance garnie d'une colonne de silice greffée C₁₈ et avec un éluant méthanol/eau 80120, compris entre 16 et 18 min.

Les substances biologiquement actives sont encore caractérisées à l'issue des étapes de purification, et lorsque les épuisements ont été effectués à l'acétone, par les données analytiques suivantes :
- un spectre UV dans le méthanol, présentant plusieurs bandes d'absorption situées à 202 nm avec un épaulement à 228 nm, entre 260 et 270 nm, entre 400 et 460 nm, et, à 630 nm.
- une chromatographie sur couche mince (CCM) présentant, après révélation chimique (anisaldéhyde sulfurique, vanilline sulfurique, phosphomolybdate, acide sulfurique éthanolique), des temps de rétention frontale de 0,15, 0,90 et 0,95.

Les substances extraites avec les solvants organiques sont souvent délicates à manipuler (évaporation, hydratation, risque de déflagration ...), il est donc souhaitable de les fixer sur un ou des supports solides tel qu'un polymère organique absorbant comme par exemple la cellulose, des minéraux ou des compositions minérales absorbantes ou des adsorbants (Silice colloïdale ...), des produits plus inertes comme les paraffines, les polyéthylènes glycols (PEG) , les propylène glycols, la polyvinylpyrrolidone etc...Tous les supports inertes peu oxygénés, peuvent convenir.

Les caractéristiques des substances fixées peuvent être très variables selon les conditions d'extraction des substances des plantes de la famille des Dictyotaceae la matière de fixation, les conditions de stabilisation et la matière sur laquelle est fixé le solvant d'extraction. La consistance est donc variable et la couleur peut varier du vert franc au marron.
La présente invention concerne encore les substances biologiquement actives adsorbées sur un matériau inerte minéral ou organique puis dessolvatées.

Les substances biologiquement actives selon l'invention, extraites de plantes de la famille des Dictyotaceae manifestent des propriétés pharmacologiques très intéressantes, et trouvent de ce fait un emploi en thérapeutique, en cosmétique, en alimentation, en diététique humaine et vétérinaire, en implantologie et en chirurgie osseuse ou articulaire.

Ces substances sont destinées à être utilisées sous forme de compositions ou d'implants pharmaceutiques, cosmétiques et/ou alimentaires.

Les substances biologiquement actives, extraites de plantes de la famille des Dictyotaceae, sont destinées à remanier la synthèse des éléments glycosylés de la matrice extracellulaire (MEC) des tissus animaux et humains. Ces substances stimulent la synthèse des glycosaminoglycanes (GAG) tels l'acide hyaluronique, la chondroïtine, les acides chondroïtines sulfuriques, l'acide dermatane sulfurique, l'acide héparane sulfurique, l'héparine, l'acide kératane sulfurique, la synthèse des protéoglycanes tels l'aggrécane, la décorine, le biglycane, le versicane, la fibromoduline, le fibroglycane, le syndécane, le bétaglycane, le glypicane.

Les substances biologiquement actives selon l'invention sont donc utilisées en vue de la réalisation de compositions destinées à remanier la synthèse des éléments glycosylés de la matrice extracellulaire (MEC) des tissus animaux et humains, et notamment à stimuler la synthèse des glycosaminoglycanes (GAG) et des protéoglycanes de la matrice extracellulaire (MEC) des tissus animaux et humains.

Les substances de la présente invention sont notamment utilisées en vue de la réalisation de compositions cosmétiques ou dermatologiques destinées à améliorer les synthèses glycosylées des glycosaminoglycanes et des protéoglycanes dans la peau.

Les substances de la présente invention stimulent les synthèses protéiques et les synthèses glycosidiques des cellules du tissu connectif ou du mésenchyme.
Les substances sont utilisées en vue de la réalisation de compositions topiques destinées à remodeler indirectement l'activité des cellules connectives ou mésenchymateuses par action directe sur les cellules épithéliales.
Les substances extraites de plantes de la famille des Dictyotaceae stimulent les synthèses golgiennes et membranaires des glycosaminoglycanes de la MEC et les synthèses golgiennes des protéoglycanes de la MEC.
Ainsi, ces substances accroissent par exemple la synthèse des collagènes, de l'acide chondroitine sulfurique, de l'acide dermatane sulfurique, de l'acide hyaluronique.
Ces substances sont utilisées en vue de la réalisations de compositions destinées à stimuler les synthèses golgiennes et membranaires des glycosaminoglycanes de la MEC et les synthèses golgiennes des protéoglycanes de la MEC.
Les substances de la présente invention sont actives sur les cellules de la peau (fibroblastes, kératinocytes), sur les cellules de l'os (ostéoblastes, chondrocytes), et sur les cellules du tissu articulaire non osseux (synoviocytes).
Chez l'homme, l'ingestion des substances biologiquement actives extraites des plantes de la famille des Dictyotaceae, tout comme la plante à l'état sec elle même, provoque une augmentation de la synthèse des éléments glycosylés de la MEC des tissus mésenchymateux et plus particulièrement de la peau (derme et épiderme), de l'os (cartilage, os spongieux, os trabéculaire, cartilage de conjugaison...).
Ainsi, les substances extraites de plantes de la famille des Dictyotaceae sont notamment utilisées en vue de la réalisation de compositions pharmaceutiques destinées à prévenir et/ou à traiter les lésions tissulaires de la peau, de l'os et du cartilage.
D'une manière générale, ces substances sont utilisées en vue de la réalisation de compositions destinées à prévenir et/ou à traiter les affections impliquant des éléments glycosylés de la matrice extracellulaire des tissus animaux et humains.

La demanderesse a démontré dans ses travaux que cette activité diffère de celle induite par la plupart des substances activatrices telles que l'estradiol et la vitamine D₃ (1-25 (OH)₂D₃), la vitamine C, etc... La ou les substances actives contenues dans les plantes expriment leur activité même en présence d'agents délétères comme les interleukines (par exemple IL-1), ces substances biologiquement actives ont donc une activité réparatrice.
Ces substances biologiquement actives ont également une activité indirecte sur les cellules, en agissant par l'intermédiaire de messagers tels qu'une cytokine, un facteur de croissance ou une hormone, sécrétés par la première population cellulaire atteinte par les substances actives extraites des plantes, et destinés aux cellules cibles.
L'activité des substances extraites des plantes de la famille des Dictyotaceae se retrouve exprimée sur des cellules provenant d'espèces animales appartenant à des embranchements aussi divers que les annélidés, les ascidies, les arthropodes, les mollusques, les bivalves (conchifères), les échinodermes, les oiseaux et les mammifères.
Les substances biologiquement actives extraites de plantes de la famille des Dictyotaceae sont encore utilisées en vue de la réalisation de compositions alimentaires destinées à améliorer l'élevage des arthropodes, des conchifères, ainsi que la qualité des coquilles des oeufs.

Les substances extraites de plantes de la famille des Dictyotaceae présentent également un grand intérêt en chirurgie osseuse. En effet, ces substances préservent le phénotype des ostéoblastes humains lors des greffes et peuvent être insérées avec un biomatériau implanté au voisinage d'une articulation pour régénérer l'os et les surfaces articulaires.
Les substances sont donc également utilisées en vue de la réalisation de compositions destinées à prévenir et/ou à traiter les affections articulaires.

Les substances de la présente invention sont également utilisées en vue de la réalisation de compositions à usage local destinées à prévenir et/ou à traiter les affections loco-régionales impliquant des éléments glycosylés de la matrice extracellulaire.

Les substances biologiquement actives sont encore utilisées en vue de la réalisation de compositions destinées à prévenir et/ou à traiter les affections liées à une dégénérescence de la matrice extracellulaire, ainsi qu'à régénérer le tissu cartilagineux, ou à complémenter les aliments.

La présente invention concerne encore les compositions pharmaceutiques, cosmétiques, et/ou alimentaires remaniant la synthèse des éléments glycosylés de la matrice extracellulaire des tissus animaux et humains, caractérisées en ce qu'elles renferment à titre de principe actif une ou plusieurs substances biologiquement actives extraites des plantes de la famille des Dictyotaceae, en association ou en mélange avec un excipient ou un véhicule inerte, non toxique, approprié à l'usage envisagé, et éventuellement un ou plusieurs principes actifs à action complémentaire.

Les compositions pharmaceutiques, cosmétiques et/ou alimentaires selon l'invention sont destinées à l'administration par voie digestive, parentérale, percutanée, topique ou rectale. Les compositions sont donc présentées sous forme de comprimés nus ou enrobés, de dragées, de capsules, de gélules, de pilules, de tablettes, de cachets, de sirops, de poudres à ingérer ou à usage externe, de compositions adjuvantes pour la cicatrisation postopératoire, pour les brûlures, les traumatismes ; de suppositoires ; de solutés ou de suspensions injectables conditionnés en ampoules ; de crèmes, de gels ou de pommades ; de solutions à usage percutané dans un solvant polaire pénétrant.

Les compositions à application cutanée mais à effets systémiques sont particulièrement originales puisqu'il a été démontré que dans ces conditions les substances biologiquement actives extraites de plantes de la famille des Dictyotaceae appliquées sur les kératinocytes (épiderme cutané) étaient susceptibles de stimuler la sécrétion d'un facteur en agissant sur les chondrocytes, les synoviocytes, les ostéoblastes et, sans doute, sur d'autres cellules mésenchymateuses.

La présente invention concerne également des implants, caractérisés en ce qu'ils renferment à titre de principe actif une ou plusieurs substances biologiquement actives extraites des plantes de la famille des Dictyotales, en association ou en mélange avec un excipient ou un véhicule inerte, non toxique, approprié à l'usage envisagé, et éventuellement un ou plusieurs principes actifs à action complémentaire.

Les exemples et résultats suivants illustrent l'invention. Ils ne la limitent en aucune façon.

### EXEMPLE I

### Etude de l'activité des substances extraites de plantes de la famille des Dictyotaceae.

### 1) Etude de l'activité

Une étude d'activité a été réalisée sur toute les fractions susceptibles d'être isolées lors d'une chromatographie liquide haute performance ou d'une chromatographie haute pression afin de montrer que les effets observés sont bien imputables à des substances biologiquement actives spécifiques des plantes.

Le demandeur a utilisé deux méthodes d'évaluation semi-quantitative pour étudier l'activité de ces plantes.
A partir d'une culture cellulaire ou tissulaire, on détecte le constituant de la matrice extracellulaire sélectionné soit par immunomarquage des cellules fixées grâce à son anticorps spécifique ; soit après destruction des cellules, on réalise une chromatographie sur gel ou une électrophorèse (chromatographie sous champ électrique) avec révélation et identification de la substance par anticorps spécifique.

Dans les deux cas, l'anticorps spécifique est révélé par un deuxième anticorps couplé soit à une molécule fluorescente, soit à un enzyme dont l'activité met en évidence un chromophore apporté par un substrat.

La quantification relative peut être réalisée par analyse d'image (immunofluorescence) ou par simple spectrophotométrie lors de l'emploi d'un substrat coloré (Western Blot). Lorsque l'analyse d'image est nécessaire, on utilise un analyseur BIOCOM 200 qui quantifie les résultats à partir de plusieurs clichés obtenus sur plusieurs cultures cellulaires. Dans tous les cas, les valeurs s'expriment en valeur relative (évaluation semi-quantitative) par rapport à un témoin et/ou par rapport à la qualité totale des protéines synthétisées. L'évaluation servi-quantitative permet de comparer les résultats entre eux. Néanmoins, il n'est pas possible de comparer la synthèse d'un composé à celle d'un autre en raison de l'affinité des anticorps qui, bien que spécifiques, n'est pas la même pour deux couples antigène-anticorps. Les résultats sont comparables entre eux pour un même anticorps.

On a étudié les synthèses matricielles des cellules isolées à partir de fragments de tissus prélevés lors d'interventions de chirurgie. Les cultures ont été réalisées en culture primaire monocouche et les résultats ont été confirmés par des cultures cellulaires tridimensionnelles. Les cultures de fragments tissulaires, peau, articulations, os, fournissent des résultats équivalents.

### 2) Résultats

Les résultats sont exprimés par un nombre correspondant à la valeur du signal perçu.
Dans les tableaux ci-dessous, les lettres «SBA» désignent les substances biologiquement actives extraites des plantes de la famille des Dictyotaceae.
• Acide dermatane sulfurique synthétisé par les fibroblastes humains en culture primaire monocouche :

| | moyenne | écart type |
|---|---|---|
| fibroblastes en milieu témoin | 32 | 0,54 |
| fibroblastes avec un milieu additionné de SBA | 625 | 21,87 |

• Acide hyaluronique synthétisé par les fibroblastes humains en culture primaire monocouche :

| | moyenne | écart type |
|---|---|---|
| fibroblastes en milieu témoin | 75 | 0,67 |
| fibroblastes avec un milieu additionné de SBA | 428 | 52,10 |

• Acide chondroïtine sulfurique synthétisé par les chondrocytes humains en culture primaire monocouche :

| | moyenne | écart type |
|---|---|---|
| chondrocytes témoins | 56 | 0,85 |
| chondrocytes avec SBA | 445 | 19,45 |

• Acide hyaluronique synthétisé par les chondrocytes humains en culture primaire monocouche :

| | moyenne | écart type |
|---|---|---|
| chondrocytes témoins | 58 | 17 |
| chondrocytes avec SBA | 108 | 21 |

### 3) Contrôle de l'activité

Ce contrôle a été réalisé conformément aux indications de la littérature en culture tridimensionnelle.
Ici, on a utilisé un gel d'acide alginique.
• Acide chondroïtine sulfurique synthétisé par les chondrocytes humains en culture tridimensionnelle :

| | moyenne | écart type |
|---|---|---|
| chondrocytes témoins | 37 | 7 |
| chondrocytes avec SBA | 94 | 8 |

• Acide hyaluronique synthétisé par les chondrocytes humains en culture tridimensionnelle :

| | moyenne | écart type |
|---|---|---|
| chondrocytes témoins | 48 | 36 |
| chondrocytes avec SBA | 151 | 50 |

Des résultats tout à fait similaires ont été obtenus avec des cultures d'ostéoblastes issus de prélèvement d'os sur diverses variétés de mammifères.

De plus, pour confirmer ce résultat, les substances biologiquement actives extraites de plantes de la famille des Dictyotaceae ont été appliquées à des cultures tissulaires de cartilages normaux et lésés. On a constaté que le cartilage lésé était pauvre en synthèse et, dans ses réserves en glycosaminoglycanes du type chondroïtine sulfate et acide hyaluronique. Le cartilage lésé traité par les substances biologiquement actives, extraites de plantes de la famille des Dictyotaceae, a présenté des taux de synthèse supérieurs au témoin sain, non traité par les substances actives.

### 4) Action pharmacologique des substances biologiquement actives

L'action pharmacologique des substances biologiquement actives extraites des plantes de la famille des Dictyotales est objectivée par :
**a)** Les activités réparatrices des substances en présence d'un agent inhibiteur délétère de la matrice extra cellulaire.
**b)** L'action indirecte des substances via un messager tel qu'une cytokine, un facteur de croissance ou une hormone.

Aujourd'hui, la littérature s'accorde à décrire la plupart des phénomènes arthrosiques comme consécutifs à une dégénérescence des surfaces articulaires.

### a) Activités réparatrices

L'activité thérapeutique d'une substance s'envisage à travers la possibilité de répondre à l'action délétère d'un intervenant biologique responsable du processus pathologique. L'interleukine 1 (IL-1) est l'intervenant physiologique majoritaire.

C'est pourquoi, on a évalué l'activité des substances biologiquement actives extraites des plantes de la famille des Dictyotaceae sur la production des glycosaminoglycanes élaborés par les cellules mésenchymateuses cultivées en présence d'IL-1.

### • Résultats des cultures en présence d'IL-1

• Acide hyaluronique synthétisé par les fibroblastes humains en culture monocouche :

| | moyenne | écart type |
|---|---|---|
| Fibroblastes cultivés en présence d'IL-1 | 36 | 9 |
| Fibroblastes cultivés en présence d'IL-1 et de SBA | 451 | 50 |

• Acide dermatane sulfurique ou acide chondroïtine sulfurique synthétisés par les fibroblastes humains en culture monocouche :

| | moyenne | écart type |
|---|---|---|
| Fibroblastes cultivés en présence d'IL-1 | 14 | 9 |
| Fibroblastes cultivés en présence d'IL-1 et de SBA | 632 | 17 |

• Acide hyaluronique synthétisé par les ostéoblastes humains en culture monocouche :

| | moyenne | écart type |
|---|---|---|
| Ostéoblastes cultivés en présence d'IL-1 | 38 | 1 |
| Ostéoblastes cultivés en présence d'IL-1 et de SBA | 232 | 5 |

• Acide chondroïtine sulfurique synthétisé par les ostéoblastes humains en culture monocouche :

| | moyenne | écart type |
|---|---|---|
| Ostéoblastes cultivés en présence d'IL-1 | 28 | 5 |
| Ostéoblastes cultivés en présence d'IL-1 et de SBA | 280 | 12 |

• Acide hyaluronique synthétisé par les chondrocytes humains en culture tridimensionnelle :

| | moyenne | écart type |
|---|---|---|
| Chondrocytes cultivés en présence d'IL-1 | 78 | 16 |
| Chondrocytes cultivés en présence d'IL-1et de SBA | 125 | 17 |

• Acide chondroïtine sulfurique synthétisé par les chondrocytes humains en culture tridimensionnelle :

| | moyenne | écart type |
|---|---|---|
| Chondrocytes cultivés en présence d'IL-1 | 25 | 3 |
| Chondrocytes cultivés en présence d'IL-1 et de SBA | 470 | 21 |

On a obtenu le même genre de résultats avec d'autres types cellulaires ainsi qu'en culture d'explants. L'évaluation semi-quantitative avec des explants est difficile car il faut tenir compte des plans de coupe.

### b) Action indirecte

Le Demandeur a constaté que, d'une manière surprenante, les cellules incubées avec les substances biologiquement actives (SBA) extraites de plantes de la famille des Dictyotaceae étaient susceptibles de transmettre le signal à d'autres cellules, signal que celles ci percevaient lors de la mise en contact avec les substances biologiquement actives. Ces substances biologiquement actives peuvent donc stimuler la sécrétion d'un deuxième messager. Ce messager atteint d'autres types cellulaires et stimule la synthèse des protéoglycanes.

Pour mettre en évidence ce phénomène, des essais ont été effectués de la manière suivante :

A une culture de kératinocytes (cellules de l'épiderme humain), on a ajouté dans le milieu de culture, les substances biologiquement actives. Après plusieurs heures d'incubation, les cellules sont rincées plusieurs fois afin d'éliminer le reste des substances biologiquement actives (SBA). Puis, un milieu neuf (K.E.M. ou Mac Coy par exemple) sans sérum de veau foetal, ni animal, est ajouté. Après une nouvelle incubation, ce milieu est prélevé. Le milieu conditionné ainsi obtenu est lyophilisé. Le lyophilisat est repris puis dilué au ¼ ou au ¹/₁₀ par le milieu spécifique dans lequel va s'opérer la deuxième culture (DMEM pour des fibroblastes, Mac Coy pour des ostéoblastes par exemple). Les cultures de cellules traitées par le milieu conditionné provenant des kératinocytes, augmentent la synthèse des protéoglycanes dans les mêmes rapports que si elles avaient été elles-mêmes traitées directement par les substances biologiquement actives alors que celles incubées en présence d'un milieu conditionné par les kératinocytes mais non incubés par les substances biologiquement actives n'ont pratiquement pas modifié leur synthèse.

De plus, la réponse au milieu conditionné varie, selon que la reprise du lyophilisat avant dilution se fait avec de l'alcool ou avec un milieu aqueux.

Dans certaines conditions expérimentales, les ostéoblastes répondent mieux lorsque le milieu conditionné par des kératinocytes est repris par de l'alcool, alors que le même traitement, entre d'une part les fibroblastes et, d'autre part les ostéoblastes, donne une meilleure réponse si le lyophilisat est repris par le milieu directement Cela laisse supposer que les messagers sécrétés par les cellules et donnant un même signal pour une même population, sont différents. Cet essai permet d'envisager la possibilité de modifier la synthèse des protéoglycanes de tissus plus profond que la peau, par une application d'une composition, contenant les composants actifs, sur les kératinocytes de la peau ou sur les tissus épithéliaux.

### EXEMPLE II

### Compositions

**1)** A titre d'exemple, on peut préparer un des extraits (contenant les substances biologiquement actives) suivants :

| | |
|---|---|
| plante séchée | 200 g |
| cyclo hexane | 1000 g |

ou :

| | |
|---|---|
| plante séchée | 200g |
| éthanol | 1000 g |

Fixation de l'extrait contenant les substances biologiquement actives (SBA) sur un support :

| | |
|---|---|
| solution organique | 200g |
| hydroxypropylméthylcellulose | 1000g |

la solution organique est évaporée sur le support de dérivé cellulosique. ou :

| | |
|---|---|
| solution organique | 20g |
| Polyéthylèneglycol (P.E.G) 4000 ou 6000 | 1 000 g |

la solution organique est distillée dans le PEG liquide ou solide. Les rapports solution organique/support sont très variables et sont liés à l'effet recherché (effet de masse, formulation retardée, action in situ, action systémique en général, etc...).

### 2) Compositions à usage général

La formulation de gélules et de comprimés, dans lesquels les substances actives sont extraites à l'éthanol et fixées sur un support inerte, s'est avérée être la plus simple. Pour ce faire, les substances biologiquement actives extraites à l'éthanol sont mélangées à de la cellulose microcristalline, à un agent lubrifiant comme le talc, et à un adjuvant de compression comme le béhénate de glycérol.

On réalise des gélules en utilisant l'adsorbat des substances biologiquement actives extraites à l'éthanol, fixé sur polyéthylèneglycol 4000 ou 6000 et dilué avec de la silice colloïdale type Aérosil® ou Sippernat®.

La voie transcutanée est formulée classiquement, avec ou sans réservoir, et avec des excipients classiques du type Transcutol®.

La voie injectable est envisageable avec les substances biologiquement actives purifiées mises en solution dans un liquide adapté pour l'injection en solution ou en suspension.

### Exemples de formulation :

a) Gélules à base de substances biologiquement actives (SBA) extraites de plantes de la famille des Dictyotaceae :

| | |
|---|---|
| SBA fixées sur polyéthylèneglycol 6000 | 95 % |
| Silice | 5 % |

Le remplissage des gélules se fait de la manière habituelle.
b) Comprimés à base de substances biologiquement actives (SBA) extraites de plantes de la famille des Dictyotales :

| | |
|---|---|
| SBA fixées sur cellulose | 25 % |
| Cellulose | 65% |
| Talc | 1.5% |
| Béhénate de glycérol | 8.5% |

Les compositions préférées pour l'usage pharmaceutique sont les formes administrables par voie orale comme les comprimés, les dragées, les gélules, les capsules contenant comme principe actif au moins une substance biologiquement active extraite de plantes de la famille des Dictyotaceae ou un adsorbat de substances actives sur un support inerte, en association ou en mélange avec un diluant ou un excipient inerte, non toxique, pharmaceutiquement compatible, et éventuellement un ou plusieurs principes actifs à action complémentaire.

Pour l'usage alimentaire, les substances biologiquement actives extraites de plantes de la famille des Dictyotaceae seront diluées dans un support alimentaire nutritif comme une farine, ou inerte comme de la cellulose ou une préparation riche en cellulose.

Ces compositions s'adressent notamment à l'élevage des crustacés, et par exemple des crevettes.

### Exemple : les crevettes.

| | |
|---|---|
| SBA | 12,1 |
| Dulectin | 23, 9 |
| Carbonate de Calcium | 5,0 |
| Prêle | 1,0 |
| Poudre germe de blé | 54,05 |
| Sippernat | 3,95 |

Cette formulation, administrée à la dose de 1.5 kg en un mois à un bassin de 180 000 crevettes, a permis l'amélioration de la survie à l'éclosion de 30 % et un gain de poids de l'animal à maturité de 10 % ± 2 %.
Ainsi, les substances biologiquement actives permettent d'augmenter le poids moyen des animaux et/ou de raccourcir les temps de maturation.

Une expérience similaire sur des poules pondeuses a montré que le nombre d'oeufs cassés lors du deuxième cycle de ponte était diminué de 2 %.

### 3) Compositions à usage loco-régional

L'usage d'implants (pellets : petits comprimés mis en place sous la peau) est déjà classique. Il a trouvé de larges applications dans le traitement hormonal substitutif. Il permet un traitement prolongé. La mise en place par voie chirurgicale de biomatériaux tels les polymères de synthèse (acide polylactique, acide polyacrylique), de minéraux : hydroxylapatite, phosphates de calcium, de biomatériaux : nacre, corail... imprégnés de principes actifs consistant en les substances biologiquement actives extraites de plantes de la famille des Dictyotales est envisagée et conduit sans aucun doute à des résultats intéressants sur la relance de la synthèse des protéoglycanes de la matrice extracellulaire.

### 4) Compositions topiques destinées aux applications à visée générale. dermatologique ou cosmétique

Pour l'usage dermatologique et cosmétique, on utilisera de préférence les crèmes ou les gels, dont l'utilisation dans ce domaine est la plus commode et la plus efficace. Sans être limitatives, ces formulations utilisent les émulsions de type huile dans l'eau ou eau dans l'huile. Ces émulsions sont appelées laits, crèmes de jour ou de nuit, dans certaines applications du domaine de la cosmétologie et dans les applications de la dermatologie, onguents, gels, formulations mixtes gel-crème.

A titre d'exemple, on pourra citer une formulation en émulsion ; ces formulations contiennent toutes au moins trois constituants : eau, corps gras, émulsifiants ou tensio-actifs. Bien évidemment, d'autres substances comme des colorants, des agents de texture, des principes actifs associés ou non, des conservateurs, des stabilisants de pH, d'oxydation, peuvent intervenir dans la formule finale. Les onguents sont des formulations ne regroupant que des excipients lipophiles. Les gels sont des compositions ayant un aspect diaphane dans lesquelles l'eau ou parfois des composants lipophiles (ou silicones) sont à l'état de colloïdes ou en suspension colloïdale.

La concentration en substances biologiquement actives (SBA) peut varier dans de larges proportions, comprises entre 0.01% et 20 % de la masse de l'émulsion, 0.01 % étant la dose seuil. Il peut être envisagé également d'utiliser des formes encapsulées de substances biologiquement actives (liposomes, nanosphères, nanocapsules,...) car celles-ci sont susceptibles d'augmenter l'activité. Avec 20 % de principe actif, il est nécessaire de modifier de manière considérable la formulation, l'activité étant sans doute en plateau.

Les formes modernes de formulation font également appel à des gels. Beaucoup de ceux-ci sont formulés à l'aide de polymères dérivés de l'acide acrylique décrits sous le nom de Carbomères. D'autres types de molécules peuvent donner des gels comme la cellulose et ses dérivés, les silices colloïdales, les polymères d'acide uronique, les mannanes, galactomannanes, les gommes xanthanes, etc...

### Exemple : Crème huile dans l'eau renfermant des substances actives extraites à l'éthanol préalablement amenées à sec,

| | |
|---|---|
| Téfose 2000® | 11 |
| Cire de lanoline | 3,5 |
| Géléol® | 3,5 |
| Eau | 76,7 |
| Conservateur | qs |
| SBA (hydrosoluble) | 5,0 |
| Parfum | 0,3 |
| SBA sous forme vésiculaire (liposomes...) | 0,5 |
| Carbomère | 0,5 |
| Solution de Na OH (10 %) | QSpH 6,5 |
| Eau | QSP 100ml |
| Conservateur, colorant | |

Il est évidemment possible d'envisager d'autres formulations dans les applications topiques, pharmaceutiques et cosmétiques, comme les éponges, les timbres à effet local, les poudres, les batonnets (stick et rouge à lèvre),...

## Revendications

1. Procédé de purification d'un extrait organique sec de plantes de la famille des Dictyotaceae **caractérisé par** une ou plusieurs étapes de purification réalisées successivement par affrontement liquide/liquide, par chromatographie sur colonne basse pression ou haute pression, par chromatographie liquide haute performance, pour obtenir des substances biologiquement actives présentant les caractéristiques suivantes :
- des temps de rétention, en chromatographie liquide haute performance semi-préparative sur le support silice greffé diol et avec un solvant éluant hexane/isopropanol 92/8, compris entre 20 et 25 min et 35 et 40 min, lorsque les épuisements ont été effectué à l'acétone,
- un temps de rétention, en chromatographie liquide haute performance semi-préparative sur le support silice greffée C₁₈ et avec un solvant éluant acétonitrile/eau 85/15, compris entre 15 et 17 min, lorsque les épuisements ont été effectué à l'acétone,
- des temps de rétention, en chromatographie liquide haute performance semi-préparative sur le support silice greffée C₁₈ et avec un solvant éluant acétonitrile/eau/acide acétique 85/15/0,05, compris entre 17 et 20 min et 30 et 40 min, lorsque les épuisements ont été effectué à l'acétone,
- un temps de rétention, en chromatographie liquide haute pression sur colonne de silice normale et avec un solvant éluant heptane/éther (90/10), compris entre 6,9 min et 8,5 min, lorsque les épuisements ont été effectué à l'éthanol,
- un temps de rétention, en chromatographie liquide haute performance garnie d'une colonne de silice greffée C₁₈ et avec un solvant éluant méthanol/eau 80/20, compris entre 16 et 18 minutes, lorsque les épuisements ont été effectué à l'éthanol.

2. Substances biologiquement actives susceptibles d'être obtenues selon la revendication 1, **caractérisées en ce qu'**elles sont de structure polycyclique, qu'elles comportent une chaîne latérale ayant de 4 à 10 atomes de carbone en ligne droite ou ramifiée et une ou deux doubles liaisons.

3. Utilisation des substances biologiquement actives susceptibles d'être obtenues selon la revendication 1, en vue de la réalisation de compositions pharmaceutiques, cosmétiques et/ou alimentaires destinées à traiter les affections loco-régionales, les cicatrices postopératoires, les brûlures et les traumatismes impliquant des éléments glycosylés de la matrice extracellulaire de tissus animaux ou humains, en association ou en mélange avec un excipient ou un véhicule inerte, non toxique, approprié à l'usage envisagé, et éventuellement un ou plusieurs principes actifs à action complémentaire.

## Patentansprüche

1. Verfahren zur Reinigung eines organischen Trockenextrakts aus Pflanzen der Familie der Dictyotaceae, **gekennzeichnet durch** einen oder mehrere Reinigungsschritte, die nacheinander **durch** Flüssig/Flüssig-Extraktion, **durch** Niederdruck- oder Hochdruck-Säulenchromatographie bzw. **durch** Hochleistungs-Flüssigchromatographie durchgeführt werden, um biologisch aktive Substanzen mit folgenden Merkmalen zu erhalten :
- Retentionszeiten bei halbpräparativer Hochleistungs-Flüssigchromatographie auf einem Diol-modifizierten Kieselgel-Trägermaterial und mit einem Elutionsmittel Hexan/Isopropanol 92/8 zwischen 20 und 25 min und 35 und 40 min, wenn die Auszüge mit Aceton durchgeführt wurden,
- eine Retentionszeit bei halbpräparativer Hochleistungs-Flüssigchromatographie auf einem C₁₈-modifizierten Kieselgel-Trägermaterial und mit einem Elutionsmittel Acetonitril/Wasser 85/15 zwischen 15 und 17 min, wenn die Auszüge mit Aceton durchgeführt wurden,
- Retentionszeiten bei halbpräparativer Hochleistungs-Flüssigchromatographie auf einem C₁₈-modifizierten Kieselgel-Trägermaterial und mit einem Elutionsmittel Acetonitril/Wasser/Essigsäure 85/15/0,05 zwischen 17 und 20 min und 30 und 40 min, wenn die Auszüge mit Aceton durchgeführt wurden,
- eine Retentionszeit bei Hochdruck-Flüssigchromatographie auf normaler Kieselgelsäule und mit einem Elutionsmittel Heptan/Ether 90/10 zwischen 6,9 min und 8,5 min, wenn die Auszüge mit Ethanol durchgeführt wurden,
- eine Retentionszeit bei Hochleistungs-Flüssigchromatographie mit einer C₁₈modifizierten Kieselgelsäule und mit einem Elutionsmittel Methanol/Wasser 80/20 zwischen 16 und 18 min, wenn die Auszüge mit Ethanol durchgerührt wurden

2. Biologisch aktive Substanzen, die nach Anspruch 1 gewonnen werden können, **dadurch gekennzeichnet, dass** sie von polyzyklischer Struktur sind, dass sie eine Seitenkette mit 4 bis 10 Kohlenstoffatomen in gerader oder verzweigter Linie und eine oder Zwei Doppelbindungen aufweisen.

3. Verwendung der biologisch aktiven Substanzen, die nach Anspruch 1 gewonnen werden können, zur Bildung von pharmazeutischen, kosmetischen und/oder Lebensmittel-Zusammensetzungen, die dazu bestimmt sind, Lokal-regionale Affektionen, postoperative Narben, Verbrennungen und Verletzungen zu behandeln, bei denen glycosylierte Elemente der extrazellulären Matrix tierischer oder menschlicher Gewebe eine Rolle spielen, in Verbindung oder germischt mit einem nicht toxischen, inerten Exzipient oder Träger, der für die beabsichtigte Verwendung geeignet ist, und eventuell einem oder mehreren Wirkstoffen mit komplementärer Wirkung.

## Claims

1. A process for purifying a dry organic extract of plants from the family of Dictyotaceae **characterized by** one or several steps of purification successively performed by liquid/liquid chromatography on column of low pressure or high pressure, by liquid chromatography high performance to obtain active biological substances showing the following features:
- retention times in semi-preparative high performance liquid chromatography (HPLC) on the support grafted silica/diol and with an eluating solvent hexane/isopropanol 92/8, ranging from 20 and 25 min and from 35 to 40 min when the extractions have been performed with acetone
- a retention in semi-preparative high performances liquid chromatography on the support grafted silica C18 using as eluating an eluating solvent acetonitrile/water/ 85/15, ranging from 15 to 17 min, extractions have been performed with acetone
- retention times in a semi-preparative high performance liquid chromatography on the support grafted silica C18 as the eluating solvent acetonitrite/water/acetic acid 85/15/0,05 ranging from 17 to 20 minutes and from 30 to 40 min when the extractions have been performed with acetone
- a retention time in high pressure liquid chromatography on a column of normal silica using as the eluting solvent heptane/ether (90/10), ranging from 6.9 min to 8,5 min, when the extractions have been performed with ethanol.
- a retention time in high performance liquid chromatography filled with a column of silica grafted with C18 and using an eluating solvent methanol/water 80/20, ranging from 16 to 18 minutes when the extractions are performed with ethanol

2. Biologically-active substances which may be obtained according to claim 1, **characterized in that** they are of polycyclic structure, they include a side chain having from 4 to 10 carbon atoms in a straight or branched chain and one or two double bonds.

3. Use of the biologically-active substances which may be obtained according to claim 1, with the ain of achieving pharmaceutical cosmetic and/or alimentary compositions intended to treat loco-regional affections, post-surgery cicatrices, burns and traumatism involving the glycosylated elements of the extra cellular matrix of the animal or human tissues, in combination or admixture with an inert non toxic carrier or vehicle suitable for the contemplated use, and optionally one or several active ingredients with complementary activity.
